(19) Europäisches Patentamt
European Patent Office
Office européen des brevets

(11) Veröffentlichungsnummer: **0 162 216**
**A1**

(12) # EUROPÄISCHE PATENTANMELDUNG

(21) Anmeldenummer: **85102930.6**

(22) Anmeldetag: **14.03.85**

(51) Int. Cl.[4]: **C 07 D 249/06**
C 07 D 249/16, C 07 D 491/044
G 03 G 5/06

(30) Priorität: **23.03.84 DE 3410700**

(43) Veröffentlichungstag der Anmeldung:
**27.11.85 Patentblatt 85/48**

(84) Benannte Vertragsstaaten:
**CH DE FR GB IT LI**

(71) Anmelder: **BASF Aktiengesellschaft**
**Carl-Bosch-Strasse 38**
**D-6700 Ludwigshafen(DE)**

(72) Erfinder: **Albert, Bernhard, Dr.**
**Eichenstrasse 60**
**D-6700 Ludwigshafen(DE)**

(72) Erfinder: **Hoffmann, Gerhard, Dr.**
**Pappelstrasse 22**
**D-6701 Otterstadt(DE)**

(72) Erfinder: **Neumann, Peter, Dr.**
**Franz-Schubert-Strasse 1**
**D-6908 Wiesloch(DE)**

(54) **2,4,5-Triphenyl-v-triazole und deren Verwendung.**

(57) 2,4,5-Triphenyltriazole der Formeln

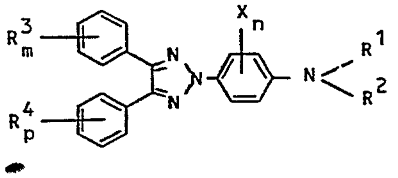

(I)

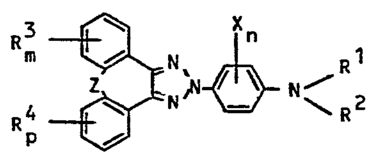

(II)

in denen $R^1$ und $R^2$ unabhängig voneinander für Alkyl, Phenalkyl, gegebenenfalls substituiertes Phenyl oder

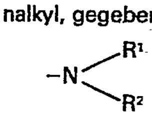

für einen gesättigten 5- oder 6-gliedrigen heterocyclischen Ring, X für Alkyl, Halogen oder Phenyl, $R^3$ und $R^4$ unabhängig voneinander für Chlor, Brom, Alkyl, Alkoxyalkyl, Alkoxy, Alkoxyalkoxy, Alkylthio, Phenyl, Phenoxy, Phenylthio oder Phenalkoxy, wobei die Phenylreste gegebenenfalls substituiert sind, oder für

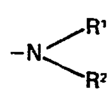

./...

EP 0 162 216 A1

n für 0, 1 oder 2, m und p jeweils für 0, 1, 2 oder 3 und Z für eine chemische Bindung oder für $>CH_2$, >0, >S oder $>N\text{-}R^1$ stehen.

Die Verbindungen (I) und (II) sind hervorragend als Ladungsträger transportierende Verbindungen in elektrophotographischen Aufzeichnungsmaterialien geeignet.

## 2,4,5-Triphenyl-v-triazole und deren Verwendung

Die Erfindung betrifft neue 2,4,5-Triphenyltriazole und deren Verwendung als Ladungsträger transportierende Verbindungen in elektrophotographischen Aufzeichnungsmaterialien.

Elektrophotographische Verfahren, dafür benötigte Materialien und verschiedene Varianten für den Aufbau von Aufzeichnungsmaterialien sind bekannt. Vorteilhaft für den Einsatz im Reproduktionssektor sind Materialien aus polymeren Bindemitteln, die an die speziellen Anforderungen des jeweiligen Einsatzgebietes angepaßt werden können, aus niedermolekularen organischen Verbindungen, die in den Bindemitteln auch in höheren Konzentrationen löslich und zu einem Transport von Ladungsträgern des elektrischen Stromes befähigt sind, sowie Verbindungen, insbesondere Farbstoffe oder Pigmente, die durch Absorption des bildmäßig eingestrahlten, aktinischen Lichtes Ladungsträger des elektrischen Stromes erzeugen und diese unter Mithilfe des von außen durch die elektrostatische Oberflächenladung aufgeprägten elektrischen Feldes auf die Ladung transportierenden Verbindungen übertragen können. Diese Ladungsträger erzeugenden Verbindungen können je nach Einsatzgebiet des Aufzeichnungsmaterials als eigene Schicht innerhalb einer Kompositstruktur eingebracht werden (DE-OS 22 20 408) oder in Form monodispers gelöster Farbstoffmoleküle in der Mischung aus Bindemittel und Ladungsträger transportierenden Verbindungen vorhanden sein (DE-PS 10 58 836). Das in der DE-OS 22 20 408 beschriebene mehrlagige elektrophotographische Aufzeichnungsmaterial besteht aus einem elektrisch leitfähigen Trägermaterial, einer ersten, Farbstoff enthaltenden, etwa 0,005 bis 2 µm dicken, durch Belichtung mit aktinischen Licht Ladungsträger des elektrischen Stromes erzeugenden Schicht und einer zweiten Schicht aus im Dunkeln isolierenden, organischen Materialien mit mindestens einer Ladungen transportierenden Verbindung.

Es ist auch bekannt, photohalbleitende organische Verbindungen zur Herstellung von elektrophotographischen Druckformen und insbesondere elektrophotographischen Offsetdruckformen zu verwenden (vgl. DE-PS 11 17 391 und 11 20 875, DE-AS 15 22 497 und 27 26 116).

Die gestiegenen Anforderungen an Reproduktionssysteme verlangen eine Vielzahl von Aufzeichnungsmaterialien und -systemen, um für spezielle Probleme optimale Lösungen aussuchen zu können. Gewünscht ist eine hohe Lichtempfindlichkeit, eine gute Auflösung und gute Betonerung. Die oft beanstandete ungenügende Betonerung, die auf eine ungünstige Feldstärkedifferenzierung zwischen belichteten und unbelichteten Flächen hinweist,

ist hierbei oft auf eine zu hohe Dunkelleitfähigkeit des Aufzeichnungsmaterials im beladenen Zustand zurückzuführen, so daß eine ungenügende Oberflächenladungsdichte vor der bildmäßigen Belichtung mit aktinischem Licht vorliegt.

Der vorliegenden Erfindung lag die Aufgabe zugrunde, neue Ladungsträger transportierende Verbindungen für elektrophotographische Aufzeichnungsmaterialien, insbesondere für die Herstellung von elektrophotographischen Druckformen wie Offsetdruckformen zu entwickeln, die bei hoher Lichtempfindlichkeit, guter Auflösung und Verarbeitung gleichzeitig ein geringes Dunkelleitvermögen aufweisen.

Es wurde nun gefunden, daß man verbesserte elektrophotographische Aufzeichnungsmaterialien mit elektrisch leitenden Trägern, Ladungsträgern erzeugenden Verbindungen bzw. Sensibilisatoren und Ladungsträger transportierenden Verbindungen erhält, wenn diese Materialien als Ladungsträger transportierende Verbindungen neue 2,4,5-Triphenyl-v-triazole der Formeln (I) oder (II)

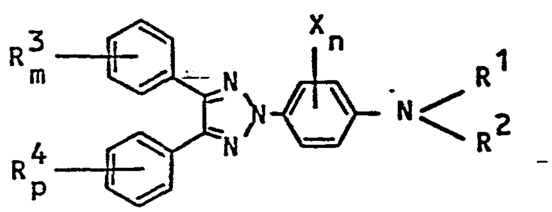

(I)

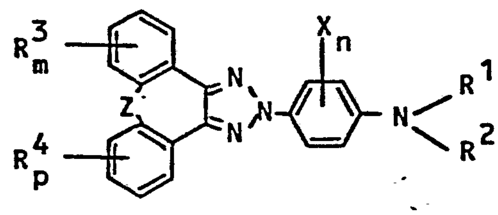

(II)

enthalten, worin

$R^1$ und $R^2$ unabhängig voneinander für $C_1$- bis $C_4$-Alkyl, Phenalkyl mit insgesamt 7 bis 10 C-Atomen oder für gegebenenfalls durch Chlor, Brom, $C_1$- bis $C_4$-Alkyl oder $C_1$- bis $C_4$-Alkoxy substituiertes Phenyl oder $-N<^{R^1}_{R^2}$ für den Rest eines gesättigten 5- oder 6-gliedrigen heterocyclischen Ringes,

X für $C_1$- bis $C_4$-Alkyl, Halogen oder Phenyl,

$R^3$ und $R^4$ unabhängig voneinander für Chlor, Brom, $C_1$- bis $C_4$-Alkyl, $C_1$- bis $C_6$-Alkoxy, $C_1$- bis $C_8$-Alkoxy-$C_2$- oder -$C_3$-alkyl, $C_1$- bis $C_8$-Alkoxy-$C_2$- oder $C_3$-alkoxy, $C_1$- bis $C_{12}$-Alkylthio, Phenyl, Phenoxy, Phenylthio oder $C_7$- bis $C_{10}$-Phenylalkoxy, wobei die Phenylgruppen gegebenenfalls durch Brom, Chlor, $C_1$- bis $C_4$-Alkyl oder $C_1$- bis $C_4$-Alkoxy, substituiert sind oder für $-N<^{R^1}_{R^2}$, worin $R^1$, $R^2$ oder $-N<^{R^1}_{R^2}$ die oben angegebene Bedeutung haben,

n für 0, 1 oder 2,
m und p unabhängig voneinander für 0, 1, 2 oder 3 und
Z für eine chemische Bindung, für $>CH_2$, $>O$, $>N-R^1$ oder $>S$ stehen.

Die elektrophotographischen Aufzeichnungsmaterialien, welche Triphenyltriazole gemäß der vorliegenden Erfindung enthalten, zeichnen sich durch eine Kombination sehr guter Eigenschaften, insbesondere durch hohe Photoleitfähigkeit bei gleichzeitig sehr niedriger Dunkelleitfähigkeit aus, weshalb die Schichten für die Kopiertechnik sehr geeignet sind. Deutliche Vorteile weisen sie bei der Verwendung für die Herstellung von elektrophotographischen Druckformen auf und genügen hierbei hohen Ansprüchen im Hinblick auf das Auflösungsvermögen und die Druckauflage.

Als Substituenten $R^1$ und $R^2$ kommen für (I) und (II) z.B. im einzelnen in Betracht:

$C_1$- bis $C_4$-Alkyl: Methyl, Ethyl, n- und i-Propyl, n-Butyl, Isobutyl;

gegebenenfalls substituiertes Phenyl: Phenyl, 2-, 3- und 4-Tolyl, 2-, 3- und 4-Methoxyphenyl, 2-, 3- und 4-Ethoxyphenyl, 2-, 3- und 4-Chlorphenyl, 2-, 3- und 4-Bromphenyl, 3- und Ethylphenyl, 3- und 4-Isopropylphenyl, 3- und 4-sec.-Butylphenyl, Phenalkyl: Benzyl, 2- und 1-Ethylphenyl, 2- und 3-Propylphenyl, Butylphenyl.

Für $-N<^{R^1}_{R^2}$ sind als Reste von gesättigten 5- und 6-gliedrigen heterocyclischen Ringen z.B. solche des Pyrrolidins, Piperidins, Morpholins, Thiomorpholins, Thiomorpholin-S-dioxids und N-$C_1$- bis $C_4$-Alkyl- und N-$C_2$- bis $C_4$-Hydroxyalkylpiperazins, wie N-Methyl-, N-Ethyl-, N-Butyl-, N-(ß-Hydroxyethyl)-piperazin und N-(ß-Hydroxypropyl)-piperazin zu nennen.

Für $R^1$ und $R^2$ sind Methyl, Ethyl, Phenyl, Benzyl und für $-N<^{R^1}_{R^2}$ Reste des Morpholins, Piperidins, Pyrrolidins und N-$C_1$- bis -$C_4$-Alkylpiperazins bevorzugt.

Für X sind im einzelnen z.B. zu nennen: $C_1$- bis $C_4$-Alkyl: Methyl, Ethyl, n- und i-Propyl, n- und i-Butyl;
Halogen steht für Brom, Fluor oder vorzugsweise Chlor;
Phenyl,
n ist 1 oder 2, vorzugsweise 0 (null).

Für $R^3$ und $R^4$ kommen z.B. im einzelnen in Betracht:

a) Wasserstoff, Brom oder Chlor;

b1) $C_1$- bis $C_4$-Alkyl: Methyl, Ethyl, n- oder i-Propyl und n- oder i-Butyl;

b2) $C_1$- bis $C_8$-Alkoxy-$C_2$- oder $C_3$-alkyl; 2-Methoxyethyl, 2-Ethoxyethyl, 2-Propoxyethyl, 2-n-Butoxyethyl, 2-n-Octoxyethyl, 2-(2'-Ethylhexoxy)-ethyl, 3-Methoxypropyl, 3-Ethoxypropyl, 3-Butoxypropyl, 3-(2'-Ethylhexoxy)-propyl;

c1) $C_1$- bis $C_6$-Alkoxy: Methoxy, Ethoxy, n- und i-Propoxy, Butoxy, Pentoxy, Hexoxy;

c2) $C_1$- bis $C_8$-Alkoxy-$C_2$- oder $C_3$-alkoxy: 2-Methoxyethoxy, 2-Ethoxyethoxy, 2-Propoxyethoxy, 2-Butoxyethoxy, 2-Hexoxyethoxy, 2-Octoxyethoxy, 2-(2'-Ethylhexoxy)-ethoxy, 3-Methoxypropopxy, 3-Ethoxypropoxy, 3-Propoxypropoxy, 3-Butoxypropoxy, 3-Hexoxypropoxy, 3-(2'-Ethylhexoxy)-propoxy, 3-Octoxypropoxy;

c3) $C_1$- bis $C_{12}$-Alkylthio: Methyl-, Ethyl-, Propyl-, Butyl-, Hexyl-, Octyl-, Decyl- und Dodecylmercapto;

c4) Phenalkoxy: Benzyloxy, 2-Phenylethoxy, 2- und 3-Phenylpropoxy;

d1) gegebenenfalls substituiertes Phenoxy: Phenoxy, 3- und 4-Chlorphenoxy, 3- und 4-Bromphenoxy, 3- und 4-Methylphenoxy, 3- und 4-Ethylphenoxy;

d2) gegebenenfalls substituiertes Phenylthio: Phenylthio, 3- und 4-Chlorphenylthio, 4-Methylthio, 3- und 4-Bromphenylthio, 3- und 4-Methoxyphenylthio, 3- und 4-Ethoxyphenylthio;

e) $-N<^{R^1}_{R^2}$, wobei $R^1$, $R^2$ oder $-N<^{R^1}_{R^2}$ die oben angegebene Bedeutung haben.

Bevorzugt sind Triazole (I) und (II), in denen $R^3$ und $R^4$ für Chlor, Brom, Methyl, Methoxy, Ethoxy, Ethyl, Methoxyethyl, Benzyl, Benzyloxy, Dimethylamino, Diethylamino oder Diphenylamino stehen.
stehen.

m und p stehen vorzugsweise für 1 oder 0 (null).

Z steht in (II) vorzugsweise für eine chemische Bindung, für $>CH_2$ oder $>O$.

Aus anwendungstechnischen Gründen sind Triphenyltriazole der Formeln III und IV

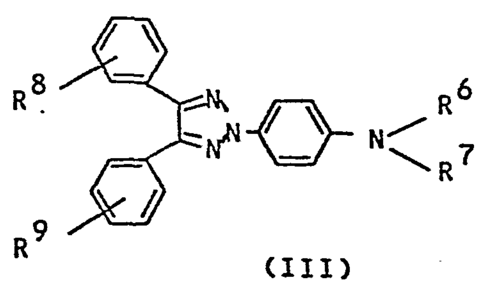

(III)

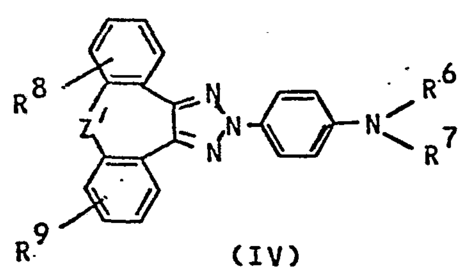

(IV)

bevorzugt. In den Formeln stehen
$R^6$ und $R^7$ unabhängig voneinander für Methyl, Ethyl, Benzyl und Phenyl und $R^8$ und $R^9$ unabhängig voneinander für
Wasserstoff, Methyl, Ethyl, Methoxy, Ethoxy, Chlor, Brom, Dimethylamino oder Diethylamino, wobei diese Substituenten in der 2-, 3- oder 4-Stellung stehen, oder für 3- oder 4-Diphenylamino und in Formel IV
Z' für eine chemische Bindung oder $\rangle O$.

Von den Verbindungen der Formel III sind besonders bevorzugt:

| | $R^6$ | $R^7$ | $R^8 = R^9$ |
|---|---|---|---|
| (III a) | $-C_2H_5$ | $-C_2H_5$ | H |
| (III b) | 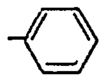 | 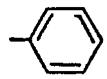 | H |

Von den Verbindungen der Formel IV sind besonders bevorzugt:

| | $R^6$ | $R^7$ | $R^8 = R^9$ | Z |
|---|---|---|---|---|
| (IV a) | $-C_2H_5$ | $-C_2H_5$ | H | chemische Bindung |
| (IV b) | 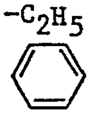 | 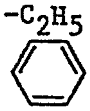 | H | " |

Die neuen Triphenyl-v-triazole (I) und (II) können nach an sich bekannten Verfahren hergestellt werden. (I) kann auf folgenden Wegen synthetisiert werden:

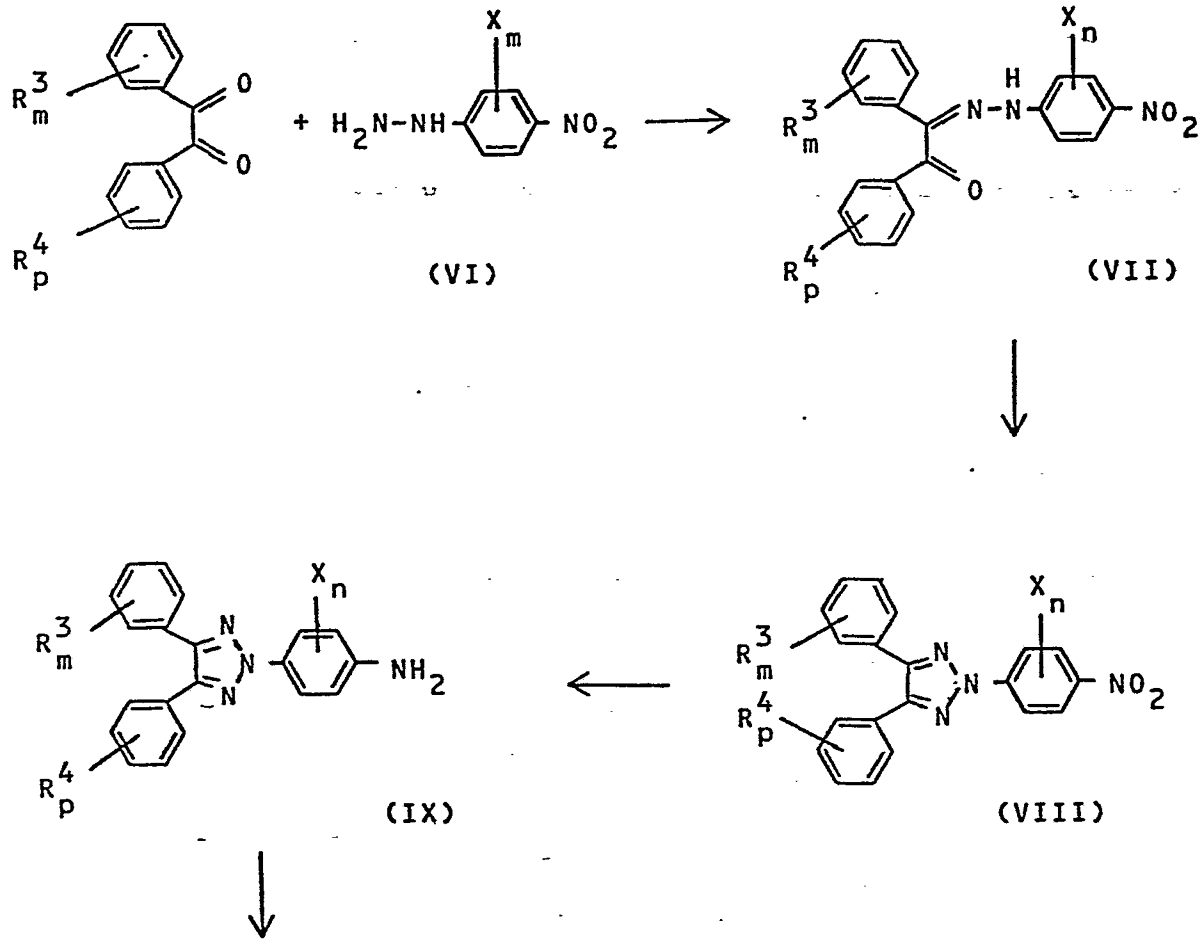

(VI) (VII) (VIII) (IX) (I)

In den Formeln haben $R^3$, $R^4$, X, m, n und p die oben angegebenen Bedeutungen.

Analog dem vorstehenden Verfahrensschema können aus der Verbindung (X) die Triazole der Formel (II) hergestellt werden:

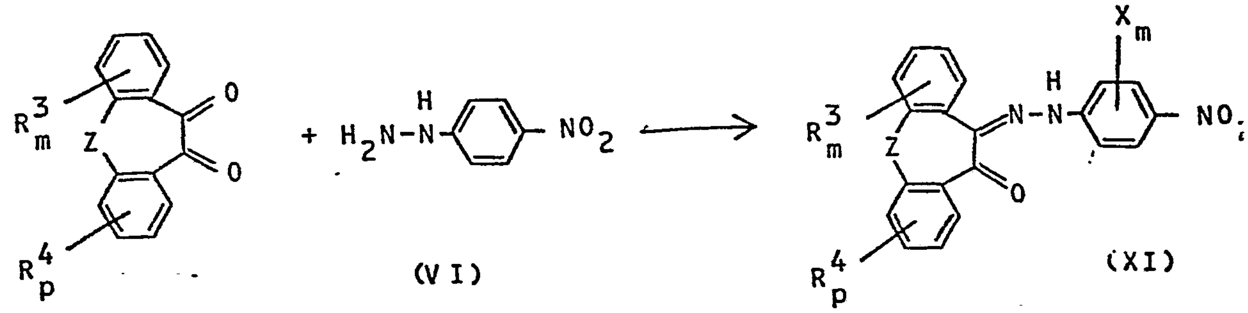

(X) (VI) (XI)

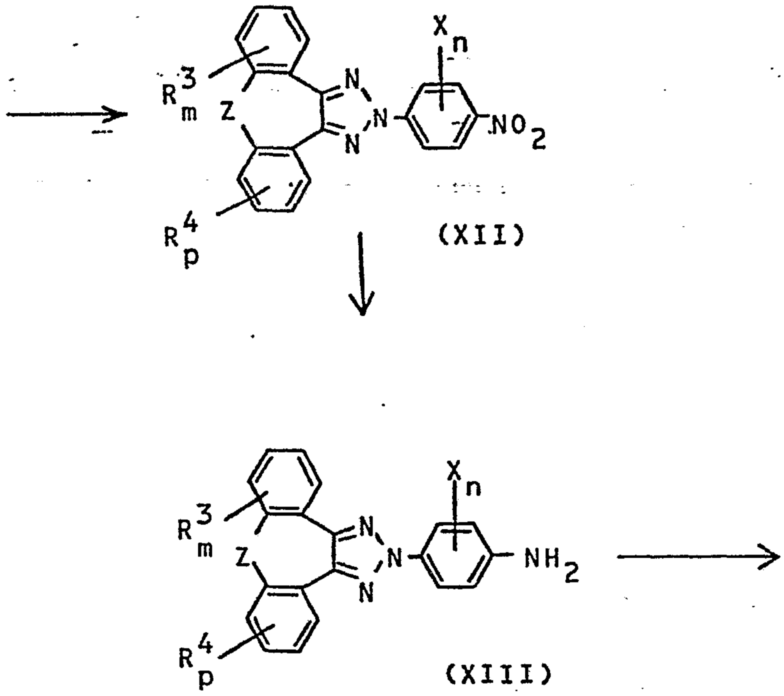

(XII)

(XIII)

(II),

wobei $R^3$, $R^4$, X, Z, m, n und p die oben angegebenen Bedeutungen haben.

Die Hydrazone (VII) und (XI) können durch Umsetzen der 4-Nitrophenylhydrazine (VI) mit dem Benzil (V) oder den Dionen (X) in Lösungsmitteln, gegebenenfalls unter Säure- oder Basenkatalyse erhalten werden. Als Lösungsmittel kommen insbesondere aliphatische Alkohole, Dimethylformamid, Eisessig oder N-Methylpyrrolidon in Betracht. Der Ringschluß von (VII) zu (VIII) bzw. von (XI) zu (XII) erfolgt durch Oxidation in Gegenwart eines Überschusses an Ammoniumsalzen.

Als Oxidationsmittel kommen hierfür z.B. Chromsäure, Alkalimetalldichromate, Wasserstoffperoxid, Blei-IV-acetat, Alkalimetallhexacyanoferrat-III, Eisen-III-chlorid und Kupfer-II-sulfat in Betracht.

Die Dichromate, Chromsäure, Wasserstoffperoxid oder Blei-IV-acetat werden vorzugsweise in sauren Lösungsmitteln wie wäßriger Essigsäure oder Propionsäure und die Hexacyanoferrate-III vorzugsweise in basischen Lösungsmitteln wie Pyridin-Wasser angewendet.

Vorzugsweise erfolgt der oxidative Ringschluß zu (VII) bzw. zu (XII) mit Kupfer-II-sulfat in Gegenwart von Ammoniumacetat in einem Pyridin-Wasser--Gemisch. Die Oxidation kann vorteilhaft auch in Methanol oder Methanol-Wasser-Gemischen mit Kupfer-II-salzen wie dem Sulfat oder dem Chlorid in Gegenwart von Ammoniumsalzen und gegebenenfalls Aminen und/oder deren Salzen erfolgen. Als Amine/Aminsalze kommen dabei vorzugsweise Mono- oder Dialkanolamine in Betracht. Der oxidative Ringschluß wird bei einer Temperatur von 70 bis 100°C, vorzugsweise 90 bis 100°C durchgeführt.

Die Reduktion der 2-Nitrophenyltriazole (VIII) und (XII) erfolgt nach an sich bekannten Verfahren.

Als Reduktionsmittel kommen Metalle wie Eisen oder Zink, Natriumsulfit, Natriumdithionit oder Hydrazin in Frage. Ebenso geeignet ist die katalytische Reduktion mit Wasserstoff in Gegenwart von Katalysatoren, wie Raney-Nickel oder Palladium in Lösungsmitteln.

Die Alkylierung der Amine (XI) und (XIII) kann nach an sich bekannten Verfahren erfolgen, z.B. mit Dialkylsulfaten, Halogenalkanen, Alkyltosylaten, Aryliodiden, vorteilhafterweise in Lösungsmitteln wie Dimethylformamid, Nitrobenzol, Alkanolen, Alkoxyalkanolen, Ketonen wie Aceton oder Cyclohexanon, Pyridin, Dimethylsulfoxid, N-Methylpyrrolidon, Tetrahydrofuran und in Gegenwart von basisch wirkenden Verbindungen wie Natriumhydrogencarbonat, Kaliumcarbonat, Natriumhydroxid oder - in geeigneten Lösungsmitteln - auch Natriumhydrid.

Im Falle der Methylierung erfolgt diese vorzugsweise nach Leuckart-Wallach mit Ameisensäure und Formaldehyd, gegebenenfalls in einem geeigneten Lösungsmittel.

Die Herstellung der Triphenyltriazole (I) und (II) wird durch die Ausführungsbeispiele weiter erläutert.

Die neuen Triphenyltriazole der Formeln (I), (II), (III) und (IV) sind hervorragend als Ladungsträger transportierende Verbindungen in elektrophotographischen Schichten geeignet. Die neuen Verbindungen können mit Vorteil sowohl in einschichtigen als auch in mehrschichtigen auf elektroleitfähige Träger aufgebrachten Aufzeichnungssystemen verwendet werden.

Für diesen Verwendungszweck sind vor allem die Triazole (III) und IV), insbesondere die Verbindungen (IIIa), (IIIb), (IVa) und (IVb) geeignet.

Geeignete einschichtige Systeme weisen bevorzugt auf einem leitfähigen Trägermaterial eine Schicht aus (a) 45 bis 75 Gewichtsteilen eines Bindemittels, (b) 30 bis 60, insbesondere 35 bis 50 Gewichtsteilen der erfindungsgemäßen Ladungsträger transportierenden Verbindungen I, (c) gegebenenfalls 5 bis 25 Gewichtsteilen eines weiteren, im wesentlichen inaktiven Bindemittels und (d) 0,05 bis 0,8 Gewichtsteilen einer bei aktinischer Belichtung Ladungsträger erzeugenden Verbindung, insbesondere eines geeigneten Farbstoffs auf. Die Schichten werden mit Vorteil aus einer ca. 6 gew.%igen Lösung in einem geeigneten organischen Lösungsmittel auf das gereinigte leitfähige Trägermaterial so aufgebracht, daß nach dem Ablüften des Lösungsmittels je nach Verwendungszweck eine Trockenschichtdicke von ca. 0,8 bis 40 µm, bei elektrophotographischen Druckformen insbesondere 0,8 bis 6 µm, resultiert.

Geeignete Mehrschichtsysteme haben auf einem elektroleitfähigen Trägermaterial z.B. (a) eine Ladungsträger erzeugende Schicht und (b) eine Ladungstransportschicht aus 30 bis 60 Gewichtsteilen mindestens einer Ladungsträger transportierenden Verbindung der Formel I, 45 bis 75 Gewichtsteilen eines organischen Bindemittels und gegebenenfalls 5 bis 25 Gewichtsteilen weiterer, die mechanischen Eigenschaften der Schicht verbessernde Zusätze. Die erste Schicht wird vorteilhaft in einer Dicke von 0,005 bis 5 µm, insbesondere 0,1 bis 0,9 µm als Lösung in einem geeigneten Lösungsmittel auf das Trägermaterial aufgetragen. Nach dem Auftrag erfolgt der Auftrag der zweiten Schicht in einer Dicke, daß nach dem Trocknen der Kompositstruktur eine Schichtdicke von 5 bis 25, insbesondere 7 bis 15 µm resultiert.

Als elektrisch leitende Träger sind prinzipiell alle leitfähigen Trägermaterialien verwendbar, soweit sie für die vorgesehene Anwendung geeignet sind. Bevorzugt sind je nach dem Anwendungsgebiet der Aufzeichnungsmaterialien Aluminium-, Zink-, Magnesium-, Kupfer- oder Mehrmetallplatten, z.B. rohe oder vorbehandelte, z.B. aufgerauhte und/oder anodisierte Aluminiumbleche, Aluminiumfolien, Polymerfilme mit metallisierter Oberfläche wie aluminiumbedampfte Polyethylentetraphthalatfilme oder auch elektrisch leitende Spezialpapiere. Träger für Druckformen haben vorteilhaft eine Dicke von 0,08 bis ca. 0,3 mm.

Die Art der geeigneten organischen Bindemittel für die Schichten richtet sich nach dem beabsichtigten Verwendungszweck der Aufzeichnungsmaterialien. Für den Kopiersektor eignen sich z.B. Celluloseether, Polyesterharze, Polyvinylchloride, Polycarbonate, Copolymere, wie Styrol-Maleinsäureanhydrid-Copolymere oder Vinylchlorid-Maleinsäureanhydrid-Copolymere oder Mischungen solcher Bindemittel. Bei ihrer Auswahl spielen ihre filmbildenden und elektrischen Eigenschaften, ihre Haftfestigkeit auf dem Trägermaterial und ihre Löslichkeitseigenschaften eine besondere Rolle. Insbesondere bei Aufzeichnungsmaterialien für die Herstellung elektrophotographischer Druckplatten und besonders bei denen für den Offsetdruck sind solche besonders geeignet, die in basischen, wäßrigen oder alkoholischen Lösungsmitteln löslich sind. Dies sind vor allem Substanzen mit alkalilöslich machenden Gruppen wie Anhydrid, Carboxyl-, Sulfonsäure-, Phenol- oder Sulfonimid-Gruppierungen. Bevorzugt sind Bindemittel, insbesondere solche mit hohen Säurenzahlen, die in basischen wäßrig-alkoholischen Lösungsmittelsystemen leicht löslich sind und ein mittleres Molekulargewicht (Gewichtsmittel) von 800 bis 80.000 und insbesondere 1.500 bis 50.000 aufweisen. Geeignet sind z.B. Copolymerisate aus Methacrylsäure und Methacrylsäureestern, besonders Copolymerisate aus Styrol und Maleinsäureanhydrid und aus Styrol, Methacrylsäure und Methacrylsäureester, soweit sie die vorstehende Löslichkeitsbedingung aufweisen. Obwohl bekanntermaßen Bindemittel mit freien Carboxylgruppen die Dunkelleitfähigkeit der elektrophotographischen Schichten in unerwünschter Weise erhöhen und dadurch zu schlechten Betonerungsergebnissen führen, lassen sich solche Bindemittel leicht an die erfindungsgemäß verwendeten Phenylindazole anpassen. So hat sich gezeigt, daß Copolymerisate aus Styrol, Maleinsäureanhydrid und Acryl- oder Methacrylsäure, die einen Anteil von einpolymerisiertem Maleinsäureanhydrid von 5 bis 50 Gew.% und einen Anteil von einpolymerisierter Acryl- oder Methacrylsäure von 5 bis 35, insbesondere 10 bis 30 Gew.% aufweisen, befriedigende elektrophotographische Schichten mit hinreichender Dunkelleitfähigkeit ergeben. Sie weisen eine hervorragende Löslichkeit in Auswaschmitteln aus 75 Gew.% Wasser, 23 Gew.% Isobutanol und 2 Gew.% Soda auf, sind aber in offsettypischem Wischwasser unlöslich.

Geeignete Ladungsträger erzeugende Verbindungen bzw. Sensibilisatoren sind z.B. für einschichtig aufgetragene Systeme, wie sie auch zur Herstellung elektrophotographischer Druckformen dienen, Farbstoffe aus der Triarylmethanreihe, Xanthenfarbstoffe und Cyaninfarbstoffe. Sehr gute Ergebnisse wurden mit den erfindungsgemäßen Verbindungen der Formel I und Rhodamin B (C.I. 45170), Rhodamin 6 G (C.I. 45160), Malachitgrün (C.I. Basic Green 4; C.I. 4200), Methylviolett (C.I. 42535) oder Kristallviolett (C.I. 42555) erhalten. Bei mehrschichtig aufgetragenen Systemen liegt der Farbstoff oder das Pigment in einer separaten Ladungsträger erzeugenden Schicht vor. Hier sind Azofarbstoffe, Phthalocyanine, Isoindolinfarbstoffe und Perylentetracarbonsäurederivate gut wirksam. Besonders gute Ergebnisse werden mit Perylen-3,4:9,10-tetracarbonsäurediimidderivaten erzielt, wie sie in den DE-OS 31 10 954 und 31 10 960 beschrieben sind.

Für die jeweilige Verwendung kann das erfindungsgemäße elektrophotographische Aufzeichnungsmaterial übliche Zusätze enthalten, z.B. Verlaufmittel

und Weichmacher in der photoleitfähigen Schicht oder Haftvermittler zwischen Träger und Schicht.

Die mit den erfindungsgemäßen Triphenyltriazolen hergestellten elektrophotographischen Aufzeichnungsmaterialien zeichnen sich durch eine Kombination sehr guter Eigenschaften, insbesondere einer hohen Photoleitfähigkeit bei gleichzeitig sehr niedriger Dunkelleitfähigkeit aus, so daß die Schichten für die Kopiertechnik sehr geeignet sind.

Deutliche Vorteile weisen diese Materialien bei der Verwendung für die Herstellung von elektrophotographischen Druckformen auf und genügen hierbei hohen Ansprüchen im Hinblick auf das Auflösungsvermögen und die Druckauflage. Aus einer sehr randscharfen Bildwiedergabe resultiert eine gute Auflösung. Durch einen hohen Ladungskontrast können auch feine Rasterpunkte in den lichten Tonwortbereichen gut wiedergegeben werden. Ferner führt die Belichtung der Schichten zu sehr geringen Restspannungen und die bei der Betonerung erhaltenen Bilder zeichnen sich durch gute Grundfreiheit in den Nichtbildbereichen aus.

Die Herstellung elektrophotographischer Offsetdruckformen erfolgt dabei wie üblich durch eine elektrostatische Aufladung des elektrophotographischen Aufzeichnungsmaterials mittels einer Hochspannungscorona, eine direkt nachfolgende bildmäßige Belichtung, die Entwicklung des vorliegenden elektrostatischen, latenten Ladungsbildes mittels eines Trocken- oder Flüssigtoners, die Fixierung des Toners durch einen nachgeschalteten Schmelzvorgang und die Entfernung der unbetonerten, photohalbleitenden Schicht mittels eines geeigneten Auswaschlösemittels. Die so erhaltene Druckform kann in bekannter Weise noch für den Offsetdruck vorbereitet werden, z.B. durch eine Hydrophilierung und Gummierung der wasserführenden Oberfläche.

Die Anwendung der Triphenyltriazole (I) und (II) wird in den Anwendungsbeispielen erläutert und belegt.

Die genannten Teile und Prozentangaben beziehen sich auf das Gewicht.

Herstellung der Triphenyltriazole

Beispiel 1

3-(4'-Diethylaminophenyl)-4,5-diphenyl-v-triazol (IIIa)

1.1

Ein Gemisch von 52,5 Teilen Benzil und 38,6 Teilen p-Nitrophenylhydrazin wird in 125 Teilen Isopropanol 4 h unter Rückfluß gekocht. Nach dem Erkalten werden die ausgefallenen Kristalle abgesaugt und mit Methanol gewaschen.
Ausbeute: 78,3 Teile der Verbindung der Formel (VII) mit $X = R^3 = R^4 = H$.
Schmp. 186 bis 188°C

1.2

Ein Gemisch aus 17,2 Teilen des nach 1.1 erhaltenen Hydrazons, 17,1 Teilen Kupfer-(II)-chlorid und 39 Teilen Ammoniumacetat wird in 100 Teilen Dimethylformamid 10 h bei 100°C gerührt. Nach dem Erkalten wird das Reaktionsgemisch in Wasser gegossen und die Fällung abfiltriert.
Ausbeute: 17,0 Teile der Verbindung der Formel (VIII) mit $X = R^3 = R^4 = H$.
Schmp. 128 bis 132°C.

1.3

Eine Lösung von 68,4 Teilen des in 1.2 erhaltenen Triazols in 400 Teilen Dimethylformamid wird mit Raney-Nickel als Katalysator bei 60°C hydriert. Nach der Beendigung der Wasserstoffaufnahme wird die Lösung filtriert und in Wasser gegossen. Der Niederschlag wird abgesaugt und mit Wasser gewaschen.
Ausbeute: 50,3 Teile der Verbindung der Formel (IX) mit $X = R^3 = R^4 = H$.
Schmp. 270 bis 272°C.

1.4

Eine Lösung von 15,6 Teilen der nach 1.3 erhaltenen Aminoverbindung in 70 Teilen N-Methylpyrrolidon wird mit 13,1 Teilen Bromethan und 10,1 Teilen Natriumhydrogencarbonat 5 h bei 100°C gerührt. Nach dem Erkalten wird filtriert und das Filtrat in Wasser gegossen. Der Niederschlag wird abgesaugt, mit Wasser gewaschen und aus Alkohol umkristallisiert.
Ausbeute: 17,7 Teile der Verbindung der Formel (IIIa).
Schmp. 114 bis 115°C.

Beispiel 2

2.1

Eine Mischung 45,9 Teilen p-Nitrophenylhydrazin und 54,6 Teilen Acenaphthochinon werden in 450 Teilen Isopropanol 2 h unter Rückfluß erhitzt. Nach dem Erkalten werden die ausgeschiedenen Kristalle abgesaugt und mit Methanol gewaschen.
Ausbeute: 91,8 Teile der Verbindung der Formel (XI) mit $X = R^3 = R^4 = H$ und Z = eine chemische Bindung.
Schmp. 258 bis 259°C.

2.2

Das Gemisch aus 79,3 Teilen des nach 2.1 erhaltenen Hydrazons, 85,3 Teile Kupfer-(II)-chlorid und 192,5 Teilen Ammoniumacetat wird in 500 Teilen Dimethylformamid 6 h auf 100°C erwärmt, nach dem Abkühlen gießt man in Wasser. Der Niederschlag wird abgesaugt und mit Wasser gewaschen.
Ausbeute: 73,5 Teile der Verbindung der Formel (XII= mit $X = R^3 = H$ und Z = eine chemische Bindung.
Schmp. 307 bis 310°C.

2.3

31,4 Teile des nach 2.2 erhaltenen Triazols werden in 300 Teilen Dimethylformamid gelöst und nach der Zugabe von Raney-Nickel bei 60°C hydriert. Nach Filtration gießt man die Lösung in Wasser und saugt den Niederschlag ab.
Ausbeute: 26,6 Teile der Verbindung der Formel (XIII) mit $X = R^3 = R^4 = H$ und Z = eine chemische Bindung.
Schmp. 262 bis 264°C.

2.4

Eine Mischung aus 11,4 Teilen der Aminoverbindung aus 2.3, 18,4 Teilen Jodbenzol, 11,1 Teilen Kaliumacetat und 0,4 Teilen Kupferpulver in 100 Teilen Nitrobenzol wird 8 h bei 120°C gerührt. Nach dem Abkühlen wird das Nitrobenzol mit Wasserdampf abgetrieben und das Umsetzungsprodukt isoliert. Das Rohprodukt wird in heißem Dimethylformamid aufgenommen und filtriert. Nach dem Erkalten wird das auskristallisierte Produkt abgesaugt und mit Methanol gewaschen.
Ausbeute: 10,1 Teile der Verbindung der Formel (IVa).
Schmp. 244 bis 245°C.

Beispiele 3 bis 12

Analog Beispiel 1 wurden die folgenden Triphenyl-v-triazole der Formel (III)

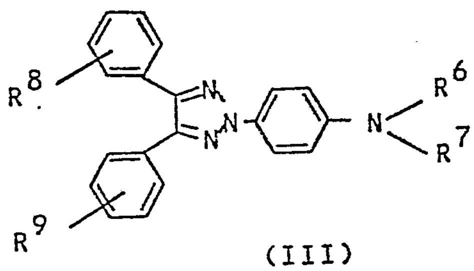

(III)

hergestellt. Die Bedeutung von $R^6$, $R^7$, $R^8$ und $R^9$ ist in der folgenden Tabelle angegeben.

Tabelle

| Beispiel | $R^6 = R^7$ | $R^8$ | $R^9$ | Schmp. [°C] |
|---|---|---|---|---|
| 3 | $-C_2H_5$ | -Cl(p) | -Cl(p) | 142-144 |
| 4 | $-C_2H_5$ | -Cl(o) | -Cl(o) | 139-142 |
| 5 | $-CH_2-$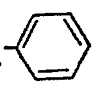 | -Cl(p) | -Cl(p) | 156-158 |
| 6 | 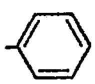 | -Cl(p) | -Cl(p) | 148-150 |
| 7 | $-CH_2-$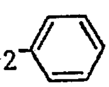 | $-OCH_3$(p) | -H | 118-121 |
| 8 | " | $-N(CH_3)_2$(p) | H | 140-143 |
| 9 | " | -Cl(o) | $-N(CH_3)_2$(p) | 61- 63 |
| 10 | 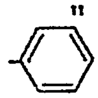 | -Cl(o) | $-N(CH_3)_2$(p) | 108-110 |
| 11 | $-CH_3$ | H | H | 148-150 |
| 12 | 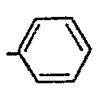 | H | H | 182-183 |

Beispiel 13 bis 15

Analog den Angaben in Beispiel 2 wurden Triazole der Formel (IVa)

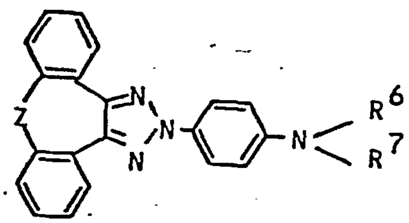

(IV a)

hergestellt. Die Bedeutung von $R^6$, $R^7$ und Z ist in der folgenden Tabelle angegeben.

| Beispiel | $R^6 = R^7$ | >Z | Schmp. [°C] |
|---|---|---|---|
| 13 | $-CH_3$ | chemische Bindung | 224-226 |
| 14 | $-CH_2-$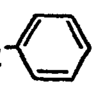 | " | 268-270 |
| 15 | 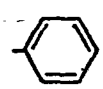 | " | 244-245 |

Anwendungsbeispiele 1 bis 4

Auf eine Polyethylenterephthalatfolie mit einer aufgedampften, leitfähigen Aluminiumschicht in einer Dicke von etwa 300 Å wird eine Schicht aus 60 Teilen eines chlorierten Perylen-3,4:9,10-tetracarbonsäurediimidbisbenzimidazols mit einem Chlorgehalt von etwa 38 % und 50 Teilen eines Copolymerisates aus Vinylchlorid, Acrylsäure und einem Maleinsäurediester in einer Dicke von etwa 0,55 µm als Ladungsträger erzeugende Schicht aufgebracht.

Auf diese Ladungsträger erzeugende Schicht wird mit Hilfe einer Lösung aus 55 Teilen eines handelsüblichen Bindemittels auf der Basis Polycarbonat mit einem Schmelzbereich von 220 bis 230°C und jeweils 40 Teilen der in der folgenden Tabelle angegebenen Triphenyltriazole in Essigsäureethylester eine Ladungstransportschicht so aufgebracht, daß nach dem Ablüften und Trocknen (30 Min. bei 80°C) eine Schichtdicke von 12 µm vorhanden ist.

An den so erhaltenen Aufzeichnungsmaterialien wurde die Halbwertsphotoempfindlichkeit in $J.m^{-2}$ ermittelt. Die Ergebnisse sind in der Tabelle I zusammengestellt.

| Anwendungsbeispiel | Triphenyltriazol aus Beispiel | Halbwertsphotoempfindlichkeit $[J.m^{-2}]$ |
|---|---|---|
| 1 | 1 | 0,35 |
| 2 | 12 | 1,15 |
| 3 | 2 | 0,42 |
| 4 | 15 | 0,55 |

Wie die Meßergebnisse zeigen, weisen die elektrophotographischen Aufzeichnungsmaterialien mit den Triphenyltriazolen gemäß der vorliegenden Erfindung eine hohe Photoleitfähigkeit auf. So zeigt z.B. die Schicht des Anwendungsbeispiels 1 im Dunkeln innerhalb von etwa 0,2 Sekunden einen Potentialabfall von 600 auf 599 Volt, während in der gleichen Schicht in der gleichen Zeit bei Belichtung mit einer Beleuchtungsstärke von 0,6 $mW.cm^{-2}$ das Potential von 600 auf 300 Volt abfällt. Die maximale Beladbarkeit ist mit größer 1.200 Volt weit über den in Kopiergeräten erforderlichen Oberflächenpotentialen (ca. 700 Volt), so daß die Schichten für die Kopiertechnik sehr geeignet sind.

Anwendungsbeispiel 5

50 Teile eines Copolymerisates aus 70 % Styrol, 24 % Acrylsäure und 6 % Maleinsäureanhydrid mit einem mittleren Molekulargewicht $M_w$ von 10.000, 50 Teile des Triphenyltriazols aus Beispiel 1 und 0,6 Teile Kristallviolett (C.I. Basic Violet 3; C.I. No. 42 555) werden mit Hilfe einer 5 %igen Lösung in Tetrahydrofuran auf eine elektrolytisch aufgerauhte und anodisierte Aluminiumfolie von 0,15 mm Dicke in einer Trockenschichtdicke von etwa 4 µm aufgebracht.

Diese Druckplatte wird nach einer Aufladung mittels einer Hochspannungscorona in einer Kamera bildmäßig 25 Sekunden belichtet. Danach wird mit einem Pulvertoner entwickelt, der bei 160°C abriebfest eingebrannt wird. Die unbetonerte Schicht mit einem Gemisch aus 0,5 % Soda, 25 % Isopropanol und 74,5 % Wasser abgewaschen, wodurch die Aluminiumoberfläche freigelegt wird. Die Lösungen werden mit einem Wattebausch über die Schicht gestrichen. Man erhält die im Offsetdruck erwünschte Differenzierung in

hydrophile und oleophile Bereiche, wobei die Trägeroberfläche die hydrophilen Bereiche liefert.

Anschließend an die Behandlung mit der alkalischen Flüssigkeit wird die Druckplatte mit Wasser nachgespült und durch Überwischen mit verdünnter Phosphorsäurelösung die Hydrophilie der Trägeroberfläche weiter erhöht. Nach Einfärben mit fetter Farbe wird auf bekannte Weise in Offsetdruckmaschinen damit gedruckt.

Patentansprüche

1. 2,4,5-Triphenyl-v-triazole der Formeln

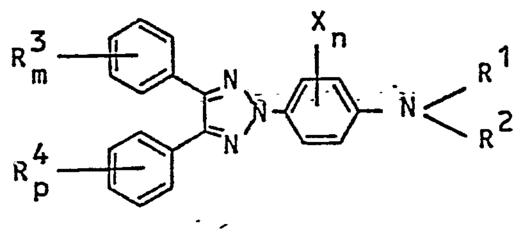

(I)

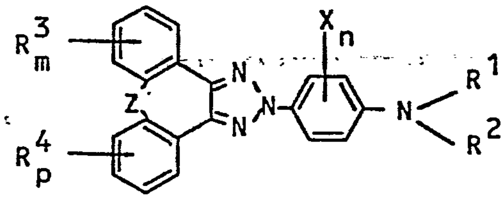

(II)

in denen

$R^1$ und $R^2$ unabhängig voneinander für $C_1$- bis $C_4$-Alkyl, Phenalkyl mit insgesamt 7 bis 10 C-Atomen oder für gegebenenfalls durch Chlor, Brom, $C_1$- bis $C_4$-Alkyl oder $C_1$- bis $C_4$-Alkoxy substituiertes Phenyl oder $-N{<}^{R^1}_{R^2}$ für den Rest eines gesättigten 5- oder 6-gliedrigen heterocyclischen Ringes,

X für $C_1$- bis $C_4$-Alkyl, Halogen oder Phenyl,

$R^3$ und $R^4$ unabhängig voneinander für Chlor, Brom, $C_1$- bis $C_4$-Alkyl, $C_1$- bis $C_8$-Alkoxy-$C_2$- oder -$C_3$-alkyl, $C_1$- bis $C_6$-Alkoxy, $C_1$- bis $C_8$-Alkoxy-$C_2$- oder -$C_3$-alkoxy, $C_1$- bis $C_{12}$-Alkylthio, für Phenyl, Phenoxy, Phenylthio oder $C_7$- bis $C_{10}$-Phenalkoxy, wobei die Phenylgruppen gegebenenfalls durch Chlor, Brom, $C_1$- bis $C_4$-Alkyl oder $C_1$- bis $C_4$-Alkoxy substituiert sind, oder für $-N{<}^{R^1}_{R^2}$, worin $R^1$, $R^2$ oder $-N{<}^{R^1}_{R^2}$ die oben angegebene Bedeutung haben,

n für 0, 1 oder 2,

m und p jeweils für 0, 1, 2 oder 3 und

Z für eine chemische Bindung oder für $>CH_2$, $>O$, $>S$ oder $>N-R^1$ stehen.

2. Triphenyltriazole gemäß Anspruch 1, dadurch gekennzeichnet, daß $R^1$ und $R^2$ unabhängig voneinander für Methyl, Ethyl, Phenyl, Benzyl oder $-N{<}^{R^1}_{R^2}$ für Morpholinyl, Piperidinyl, Pyrrolidinyl oder N-$C_1$- bis $C_4$-Alkylpiperazinyl stehen.

3. Triphenyltriazole gemäß Anspruch 1 oder 2, dadurch gekennzeichnet, daß n = 0 (null) ist.

4. Triphenyltriazole gemäß Anspruch 1, 2 oder 3, dadurch gekennzeichnet, daß $R^3$ und $R^4$ für Chlor, Brom, Methyl, Ethyl, Methoxy, Ethoxy, Methoxyethyl, Benzyl, Benzyloxy, Dimethylamino, Diethylamino oder Diphenylamino und m und p für 1 oder 0 (null) und Z für eine chemische Bindung, $>CH_2$ oder $>O$ stehen.

5. Triphenyltriazole gemäß Anspruch 1, gekennzeichnet durch die Formeln

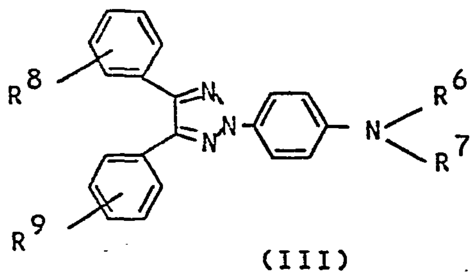
(III)

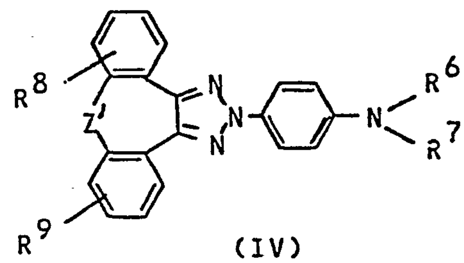
(IV)

in denen $R^6$ und $R^7$ unabhängig voneinander für Methyl, Ethyl, Benzyl oder Phenyl und $R^8$ und $R^9$ unabhängig voneinander für Wasserstoff, Methyl, Ethyl, Methoxy, Ethoxy, Chlor, Brom, Dimethylamino oder Diethylamino, wobei diese Substituenten in der 2-, 3- oder 4-Stellung stehen, oder für 3- oder 4-Diphenylamino und Z' für eine chemische Bindung oder $>O$ stehen.

6. Triphenyltriazole gemäß Anspruch 5, dadurch gekennzeichnet, daß $-N<^{R^6}_{R^7}$ für $-N(C_2H_5)_2$ oder -N-(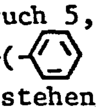)$_2$, $R^8$ und $R^9$ für Wasserstoff und Z für eine chemische Bindung stehen.

7. Verwendung der Triphenyltriazole gemäß den Ansprüchen 1 bis 6 als Ladungsträger transportierende Verbindungen in elektrophotographischen Aufzeichnungsmaterialien.

Europäisches Patentamt

# EUROPÄISCHER RECHERCHENBERICHT

0162216 Nummer der Anmeldung

EP 85 10 2930

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 093 329 (BASF AG)<br>* Ansprüche 1,2 * | 1-7 | C 07 D 249/06<br>C 07 D 249/16<br>C 07 D 491/044<br>G 03 G 5/06 |
| A | EP-A-0 093 330 (BASF AG)<br>* Ansprüche 1,2 * | 1-7 | |
| P,A | EP-A-0 131 292 (BASF AG)<br>* Ansprüche 1-7 * | 1-7 | |
| P,A | EP-A-0 116 341 (BASF AG)<br>* Ansprüche 1-9 * | 1-7 | |
| | ----- | | **RECHERCHIERTE SACHGEBIETE (Int. Cl.4)** |
| | | | C 07 D 249/00<br>C 07 D 487/00<br>C 07 D 491/00<br>C 07 D 495/00<br>G 03 G 5/00 |

Der vorliegende Recherchenbericht wurde für alle Patentansprüche erstellt.

| Recherchenort | Abschlußdatum der Recherche | Prüfer |
|---|---|---|
| BERLIN | 20-06-1985 | HASS C V F |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument